# EUROPEAN PATENT SPECIFICATION

(11) **EP 3 970 758 B1**
(45) Date of publication and mention of the grant of the patent: **07.06.2023**
(21) Application number: 21186168.7
(22) Date of filing: 16.07.2021
(51) Int. Cl.: A61L 2/14

(54) **UNIT AND PROCESS FOR STERILIZING ARTICLES SUCH AS CAPSULES, PARISONS, CONTAINERS**
EINHEIT UND VERFAHREN ZUM STERILISIEREN VON GEGENSTÄNDEN WIE KAPSELN, VORFORMLINGEN, BEHÄLTERN
UNITÉ ET PROCÉDÉ DE STÉRILISATION D'ARTICLES TELS QUE DES CAPSULES, PARAISONS, RÉCIPIENTS

(30) Priority: 22.09.2020 IT 202000022372
(43) Date of publication of application: 23.03.2022
(73) Proprietor: Gea Procomac S.p.A., 43038 Sala Baganza (PR) (IT)
(72) Inventor: ANTIGA, Simone, 43013 Langhirano (PR) (IT); COMANI, Andrea, 43038 Sala Baganza (PR) (IT)
(74) Representative: Dondi, Silvia

(56) References cited:
- EP-A1- 3 363 746
- US-A- 4 909 995
- US-A1- 2006 280 646

## Description

The present invention relates to a unit and a process for sterilizing articles such as capsules, parisons, containers. In particular, the articles referred to herein are made of thermoplastic material.

The reference sector for the present invention is the bottling of so-called "sensitive" food products, i.e., products which are particularly sensitive to bacteriological contamination and oxidation, such as isotonic beverages, juices, nectars, soft drinks, tea, milk-based beverages, coffee-based beverages, etc., for which avoiding possible microbiological contamination throughout all the packaging steps is of fundamental importance.

As is known, in a bottling line with aseptic technology it is of primary importance to control contamination in the various work stations (forming, filling, capping, etc.), thus it is essential to ensure adequate filtering of the fluids to be introduced into the controlled environment, correct management of the pressures of the various areas in order to control the path of any unwanted particles, correct monitoring of the environment, correct management and adequate quality of the cleaning and sterilization cycles of the internal parts of the machine (C.I.P., acronym for "Cleaning In Place" and S.I.P., acronym for "Sterilization In Place"), or of the external parts of the machine (C.O.P., acronym for "Cleaning Out of Place" and S.O.P., acronym for "Sterilization Out of Place").

In an aseptic bottling line, depending on the different types of technologies used, there is a need to sterilize parisons or containers formed before the filling and relative capping. There is a need to also sterilize the closures (caps or capsules) of the containers themselves.

In this context, a known solution is described in EP3363746, which relates to a plasma sterilization system of capsules advancing along a helical path. In particular, there are numerous plasma dispensers in succession above the advancement path, placed one after the other to send the plasma on the underlying capsules. The helical configuration of the path allows the capsules to tip over during the advancement thereof and thus expose different surfaces to the plasma flow.

However, the solution described has some disadvantages. First of all, it requires a large number of dispensers arranged along the path, which make the system cumbersome and expensive. Furthermore, in order to ensure a correct sterilization of the capsules, all the dispensers must be active, requiring periodic maintenance. Further, the specific helical configuration of the path increases the structural complexity of the sterilization system.

The use of plasma for sterilizing objects is already known in other industrial fields, in particular in the medical field for sterilizing surgical instruments. However, these are sterilizations performed "in batch" with plasma under vacuum.

US 2006/0280646 A1 discloses vacuum sterilization processes and devices and US 4 909 995 discloses a process and apparatus for dry sterilization of medical devices and materials.

In this context, the technical task underpinning the present invention is that of proposing a unit and a process for sterilizing articles such as capsules, parisons, containers, which obviate the drawbacks of the prior art cited above.

In particular, the object of the present invention is to propose a sterilization unit for sterilizing articles such as capsules, parisons, containers, with reduced overall size and structural complexity with respect to the known solutions, with equal sterilization effectiveness.

Another object of the present invention is to propose a sterilization unit for sterilizing articles such as capsules, parisons, containers, which requires easier and faster maintenance with respect to the known solutions. Another object of the present invention is to propose a unit and a process for sterilizing articles which can be used flexibly in different production lines and at different points of the line, regardless of the articles to be treated (capsules, parisons, containers, etc.).

The essential features of the invention are explicitly defined in the wordings of independent claims 1 and 10. Other features of the invention are defined in the wordings of the dependent claims.

The stated technical task and specified objects are substantially achieved by a sterilization unit for sterilizing articles such as capsules, parisons, containers, comprising:
- a treatment chamber having an inlet section and an outlet section for the articles;
- a passage zone for the passage of the articles having an extension inside the treatment chamber from the inlet section to the outlet section;
- plasma supply means for supplying plasma to the treatment chamber;
- a suction air barrier system operatively active on the treatment chamber so as to limit the inlet of air from the outside towards the treatment chamber and the outlet of plasma from the treatment chamber towards the outside, thus maintaining a plasma saturated environment inside the treatment chamber.

In accordance with an aspect of the invention, the sterilization unit further comprises a first opening made in the inlet section, through which the articles are supplied to the treatment chamber, and a second opening made in the outlet section, through which the treated articles exit from the treatment chamber.

The suction air barrier system is arranged so as not to interfere with the openings. In particular, the suction air barrier system is arranged outside the treatment chamber.

In accordance with an embodiment, the suction air barrier system comprises at least one suction intake.

Preferably, the suction air barrier system comprises two suction intakes, respectively arranged at the inlet section and at the outlet section of the treatment chamber.

In accordance with an embodiment, the plasma supply means comprise at least one plasma generator and a plasma distribution line for the distribution of plasma to the treatment chamber.

In particular, the plasma distribution line branches into at least two conduits for the injection of plasma into the treatment chamber.

For example, the plasma generator is a plasma torch.

In accordance with another embodiment, the plasma supply means comprise two plasma generators arranged so as to supply the treatment chamber through corresponding distribution lines.

In accordance with an aspect of the invention, the passage zone for the passage of the articles has a substantially linear extension inside the treatment chamber.

For example, the passage zone is delimited by walls so as to define a channel or a tunnel inside the treatment chamber.

In accordance with an alternative embodiment, the sterilization unit comprises a rotating carousel provided with a plurality of gripping elements for gripping the articles and arranged inside the treatment chamber.

The passage zone has an annular extension that follows a circumferential advancement path for the articles during the rotation of the rotating carousel.

In accordance with an aspect of the invention, the sterilization unit comprises a device for housing the articles inside the treatment chamber. The stated technical task and specified objects are substantially achieved by a process for sterilizing articles such as capsules, parisons, containers, comprising the steps of:
- injecting plasma into a treatment chamber;
- maintaining a plasma saturated environment inside the treatment chamber;
- providing a continuous flow of articles across the treatment chamber in order to make them pass into said plasma saturated environment and sterilize them.

In accordance with an aspect of the invention, the step of maintaining a plasma saturated environment takes place by generating a suction air barrier at least at an inlet section and an outlet section of the treatment chamber.

In accordance with an embodiment, the articles pass through the treatment chamber, describing a linear advancement path.

In accordance with another embodiment, the articles pass through the treatment chamber describing an at least partially curvilinear path.

Further features and advantages of the present invention will more fully emerge from the non-limiting description of a preferred but not exclusive embodiment of a unit and a process for sterilizing articles such as capsules, parisons, containers, as illustrated in the accompanying drawings, in which:
- figure 1 schematically illustrates a sterilization unit of articles such as capsules, parisons, containers, in accordance with an embodiment
- figure 2 illustrates another embodiment of the sterilization unit of figure 1.

With reference to the figures, reference numeral 1 denotes a sterilization unit for sterilizing articles 100 such as capsules, parisons, containers, for examples bottles made of PET.

The sterilization unit 1 comprises a treatment chamber 2 having an inlet section 3 and an outlet section 4 for the articles 100.

In particular, a first opening 13 is made in the inlet section 3 to allow the introduction of the articles 100 to be treated inside the treatment chamber 2.

A second opening 14 is made in the outlet section 4 to allow the already treated articles 100 to exit from the treatment chamber 2.

The articles 100 transit in a passage zone 20 for the passage of the articles which extends inside the treatment chamber 2 from the inlet section 3 to the outlet section 4.

The sterilization unit 1 further comprises a plasma supply means 5, 6 for supplying plasma to the treatment chamber 2.

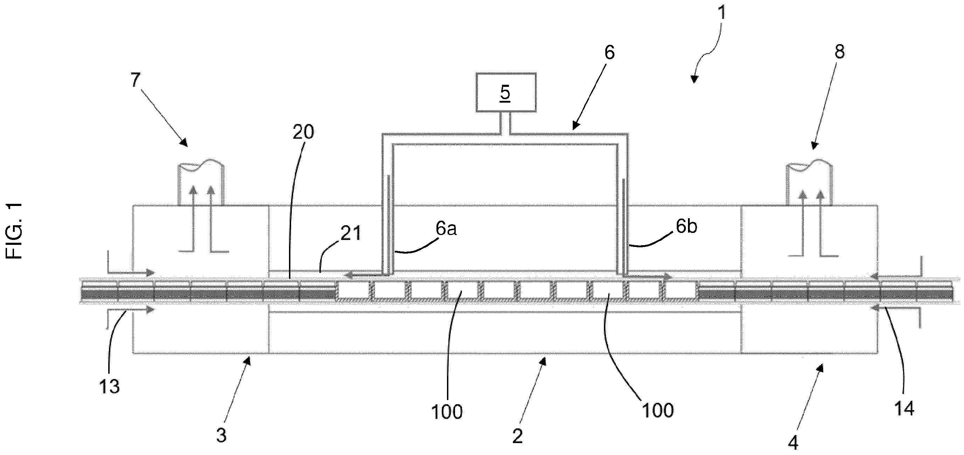

In accordance with an embodiment of the invention, illustrated in figure 1, the plasma supply means comprises a single plasma generator 5 and a plasma distribution line 6 connecting the plasma generator 5 to the treatment chamber 2.

For example, the plasma generator 5 is a plasma torch. The plasma torch is a known device and will not be described further.

In accordance with an aspect of the invention, the plasma is obtained and transported by a dry air stream to which a known amount of vaporized water is added. The water can be replaced for example by a solution containing H₂O₂.

In accordance with the embodiment of figure 1, the plasma distribution line 6 is branched into at least two conduits 6a, 6b for the injection of plasma into the treatment chamber 2.

In an embodiment not shown, the plasma distribution line 6 branches into more than two conduits for the injection of plasma into the treatment chamber 2.

There is also the possibility of making a single conduit which goes from the plasma generator 5 to the treatment chamber 2.

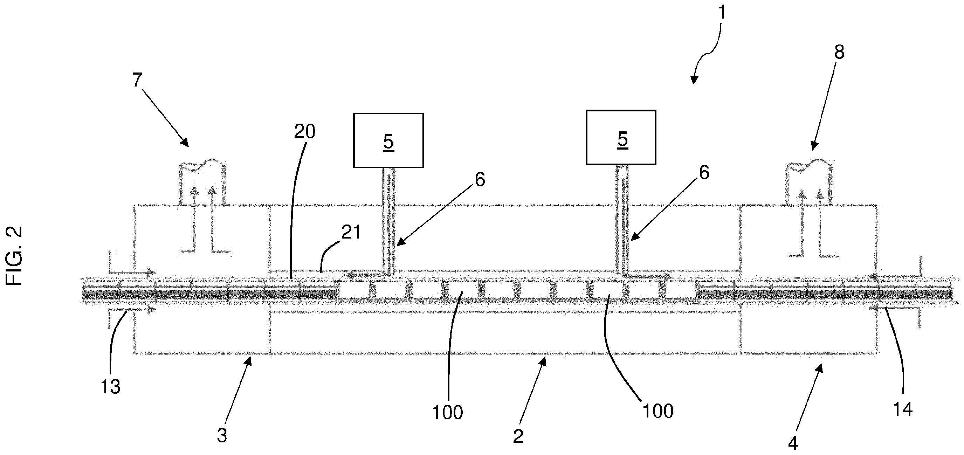

In accordance with another embodiment of the invention, illustrated in figure 2, the plasma supply means comprises two plasma generators 5, each with a corresponding distribution line 6 going to the treatment chamber 2.

The sterilization unit 1 comprises a suction air barrier system 7, 8 operatively active on the treatment chamber 2 so as to limit the inlet of air from the outside to the treatment chamber 2 and the outlet of plasma from the treatment chamber 2 to the outside. Thereby, a plasma saturated environment is maintained inside the treatment chamber 2.

In accordance with an aspect of the invention, the suction air barrier system 7, 8 is arranged so as not to interfere with the first opening 13 and with the second opening 14.

In particular, the suction air barrier system 7, 8 is arranged outside the treatment chamber 2.

In other words, the suction air barrier system 7, 8 is separated from the openings 13, 14, i.e., it is not arranged at the openings 13, 14 and does not occlude them (neither totally nor partially).

In particular, the air barrier system comprises at least one suction intake.

In accordance with the embodiments described and illustrated herein, the suction air barrier system comprises two suction intakes 7, 8 respectively arranged at the inlet section 3 and at the outlet section 4 of the treatment chamber 2.

In accordance with the embodiments described and illustrated herein, the passage zone 20 for the passage of the articles 100 has a substantially linear extension inside the treatment chamber 2 from the inlet section 3 to the outlet section 4.

Preferably, the passage zone 20 is delimited by walls defining a channel or tunnel 21 inside the treatment chamber 2.

In this case, the plasma supply means 5, 6 injects the plasma inside the channel 21 so that the articles 100 passing inside the channel 21 (i.e., in the passage zone 20) are subjected to the flow of the plasma and thus sterilized.

For example, in the case of capsules, the treatment unit 1 comprises at least one pair of straight and parallel rails arranged inside the treatment chamber 2, on which the capsules advance.

Also in the case of parisons or bottles, rails can be provided for the advancement of parisons/bottles hanging by their neck.

Alternatively, a conveyor belt for containers (resting with their bottom on the belt) can be present. The same applies to other articles.

In accordance with another embodiment (not shown), the sterilization unit 1 comprises a rotating carousel provided with a plurality of gripping elements or support compartments for the articles 100. The rotating carousel is arranged inside the treatment chamber 2. In this case, the passage zone 20 has an annular extension which follows a circumferential advancement path of the articles 100 during the rotation of the rotating carousel.

The sterilization unit 1 can also comprise a device for housing the articles 100 which is arranged inside the treatment chamber 2.

The treatment unit 2 can be interposed between two operating units of various types. For example, the treatment unit 2 can receive the capsules 100 from a capsule supply unit (e.g., from a capsule orientation and supply system) and yield the capsules to a capsuling unit.

In the case of parisons, the treatment unit 2 can receive the parisons from a warehouse or from a parison orientation and supply unit, and transfer the sterilized parisons to a heating unit.

In the case of PET bottles, the treatment unit 2 can receive the bottles from a stretching-blowing forming unit and transfer them to a filling unit.

In general, in an aseptic packaging line, the sterilization unit 1 proposed herein is suitable for defining a part of an isolating apparatus adapted to define a controlled contamination environment within which all processing and treatment operations of parisons/containers/capsules occur.

The application examples just mentioned are purely indicative and not limiting. In fact, the treatment unit 2 can also be used in other configurations or for articles other than those mentioned above.

The process for sterilizing articles such as capsules, parisons, containers is described below.

The articles 100 to be treated, for example the capsules, are supplied to the treatment unit 2 through the first opening 13.

The articles 100 transit in the passage zone 20 for the passage of the articles which extends inside the treatment chamber 2 from the inlet section 3 to the outlet section 4.

The advancement of the articles 100 can occur thanks to specifically arranged devices, e.g., star-wheels or rotating carousels, conveyor belts. Or, for example in the case of the capsules 100, the advancement occurs by gravity. In this case, the sterilization unit 1 is installed with an inclination angle so as to be able to exploit the effect of gravity to make the capsules 100 slide.

During their path - whether linear or curvilinear - the articles 100 are subjected to the flow of plasma injected by one or more generators 5 through the plasma distribution line 6.

The entire environment inside the treatment chamber 2 is plasma saturated, thus the articles 100 are sterilized uniformly.

The treated articles 100 exit from the second opening 14.

From the description given, the features of the unit and the process for sterilizing articles such as capsules, parisons, containers according to the present invention appear clear, as do the advantages thereof.

In particular, the proposed sterilization unit has a simple structure in that the articles are sterilized during their normal advancement in the treatment chamber in which a plasma saturated environment is created and maintained.

The articles can therefore be sterilized continuously by plasma without having to undergo particular movements such as to expose the different surfaces (as was the case in the solution with a helical rail and overlying dispensers).

In this case, multiple plasma dispensers following each other along the advancement path of the articles are not needed, only a plasma generator is sufficient which injects the plasma into the treatment chamber and a suction air barrier system which maintains the saturation condition. Maintenance operations are also simplified and faster, as it is sufficient to monitor the conditions of the plasma generator(s) and the suction intakes outside the treatment chamber.

## Claims

1. A sterilization unit (1) for sterilizing articles (100) such as capsules, parisons, containers, comprising:
a treatment chamber (2) having an inlet section (3) and an outlet section (4) for said articles (100);
a passage zone (20) for the passage of the articles (100) having an extension inside the treatment chamber (2) from the inlet section (3) to the outlet section (4);
plasma supply means (5, 6) for supplying plasma to said treatment chamber (2);
a suction air barrier system (7, 8) operatively active on the treatment chamber (2) so as to limit the inlet of air from the outside towards the treatment chamber (2) and the outlet of plasma from the treatment chamber (2) towards the outside, thus maintaining a plasma saturated environment inside the treatment chamber (2), said suction air barrier system (7, 8) comprising two suction intakes (7, 8) respectively arranged at the inlet section (3) and at the outlet section (4) of said treatment chamber (2), said suction air barrier system (7, 8) being arranged outside the treatment chamber (2);
a first opening (13) made in the inlet section (3) through which the articles (100) are supplied to the treatment chamber (2) and a second opening (14) made in the outlet section (4) through which the treated articles (100) exit from the treatment chamber (2), said suction air barrier system (7, 8) being arranged so as not to interfere with said openings (13, 14).

2. The sterilization unit (1) according to any one of the preceding claims, wherein said plasma supply means (5, 6) comprise at least one plasma generator (5) and a plasma distribution line (6) for the distribution of plasma to the treatment chamber (2).

3. The sterilization unit (1) according to claim 2, wherein said plasma distribution line (6) branches into at least two conduits (6a, 6b) for the injection of plasma into the treatment chamber (2).

4. The sterilization unit (1) according to claims 2 or 3, wherein said plasma generator (5) is a plasma torch.

5. The sterilization unit (1) according to any one of claim 1, wherein said plasma supply means (5, 6) comprise two plasma generators (5) arranged so as to supply said treatment chamber (2) through corresponding distribution lines (6).

6. The sterilization unit (1) according to any one of the preceding claims, wherein said passage zone (20) for the passage of the articles (100) has a substantially linear extension inside the treatment chamber (2).

7. The sterilization unit (1) according to any one of the preceding claims, wherein said passage zone (20) is delimited by walls so as to define a channel or a tunnel (21) inside the treatment chamber (2).

8. The sterilization unit (1) according to any one of claims 1 to 5, comprising a rotating carousel provided with a plurality of gripping elements for gripping the articles (100) and arranged inside the treatment chamber (2), said passage zone (20) having an annular extension that follows a circumferential advancement path for the articles (100) during the rotation of the rotating carousel.

9. The sterilization unit (1) according to any one of the preceding claims, comprising a device for housing the articles (100) inside the treatment chamber (2).

10. A process for sterilizing articles (100) such as capsules, parisons, containers in a sterilization unit (1) according to any one of the preceding claims, said process comprising the steps of:
injecting plasma into a treatment chamber (2);
maintaining a plasma saturated environment inside the treatment chamber (2), wherein the step of maintaining a plasma saturated environment takes place by generating a suction air barrier at least at an inlet section (3) and
an outlet section (4) of the treatment chamber (2);
providing a continuous flow of articles (100) across the treatment chamber (2) in order to make them pass into said plasma saturated environment and sterilize them.

11. The process for sterilizing according to claim 10, wherein the articles (100) cross the treatment chamber (2) describing a linear advancement path.

12. The process for sterilizing according to claim 10, wherein the articles (100) cross the treatment chamber (2) describing an at least partially curvilinear path.

## Patentansprüche

1. Sterilisationseinheit (1) zum Sterilisieren von Gegenständen (100) wie Kapseln, Vorformlingen, Behältern, umfassend:
eine Behandlungskammer (2) aufweisend einen Einlassabschnitt (3) und
einen Auslassabschnitt (4) für die Gegenstände (100);
eine Durchgangszone (20) für den Durchgang der Gegenstände (100) mit einer Ausdehnung innerhalb der Behandlungskammer (2) vom Einlassabschnitt (3) zum Auslassabschnitt (4);
Plasmazufuhrmittel (5, 6) zum Zuführen von Plasma zu der Behandlungskammer (2);
ein Saugluftbarrieresystem (7, 8), das auf die Behandlungskammer (2) betriebswirksam aktiv ist, um den Einlass von Luft von außen in Richtung der Behandlungskammer (2) und den Auslass von Plasma aus der Behandlungskammer (2) nach außen zu begrenzen und so eine plasmagesättigte Umgebung innerhalb der Behandlungskammer (2) aufrechtzuerhalten, wobei das Saugluftbarrieresystem (7, 8) zwei Saugeingänge (7, 8) umfasst, die jeweils am Einlassabschnitt (3) und am Auslassabschnitt (4) der Behandlungskammer (2) angeordnet sind, wobei das Saugluftbarrieresystem (7, 8) außerhalb der Behandlungskammer (2) angeordnet ist;
eine erste Öffnung (13), die in dem Einlassabschnitt (3) ausgebildet ist, durch die die Gegenstände (100) der Behandlungskammer (2) zugeführt werden, und eine zweite Öffnung (14), die in dem Auslassabschnitt (4) ausgebildet ist, durch die die behandelten Gegenstände (100) aus der Behandlungskammer (2) austreten, wobei das Saugluftbarrieresystem (7, 8) so angeordnet ist, dass es die Öffnungen (13, 14) nicht stört.

2. Sterilisationseinheit (1) nach einem der vorhergehenden Ansprüche, wobei die Plasmazufuhrmittel (5, 6) mindestens einen Plasmagenerator (5) und eine Plasmaverteilungsleitung (6) für die Verteilung von Plasma an die Behandlungskammer (2) umfassen.

3. Sterilisationseinheit (1) nach Anspruch 2, wobei sich die Plasmaverteilungsleitung (6) in mindestens zwei Leitungen (6a, 6b) für die Einspritzung von Plasma in die Behandlungskammer (2) verzweigt.

4. Sterilisationseinheit (1) nach Anspruch 2 oder 3, wobei der Plasmagenerator (5) ein Plasmabrenner ist.

5. Sterilisationseinheit (1) nach Anspruch 1, wobei die Plasmazufuhrmittel (5, 6) zwei Plasmageneratoren (5) umfassen, die so angeordnet sind, dass sie die Behandlungskammer (2) durch entsprechende Verteilungsleitungen (6) versorgen.

6. Sterilisationseinheit (1) nach einem der vorhergehenden Ansprüche, wobei die Durchgangszone (20) für den Durchgang der Gegenstände (100) eine im Wesentlichen lineare Ausdehnung innerhalb der Behandlungskammer (2) aufweist.

7. Sterilisationseinheit (1) nach einem der vorhergehenden Ansprüche, wobei die Durchgangszone (20) durch Wände begrenzt ist, um einen Kanal oder einen Tunnel (21) innerhalb der Behandlungskammer (2) zu definieren.

8. Sterilisationseinheit (1) nach einem der Ansprüche 1 bis 5, umfassend ein rotierendes Karussell, das mit einer Vielzahl von Greifelementen zum Greifen der Gegenstände (100) versehen und innerhalb der Behandlungskammer (2) angeordnet ist, wobei die Durchgangszone (20) eine ringförmige Ausdehnung aufweist, die einem Umfangsvorschubweg für die Gegenstände (100) während der Rotation des rotierenden Karussells folgt.

9. Sterilisationseinheit (1) nach einem der vorhergehenden Ansprüche, umfassend eine Vorrichtung zum Aufnehmen der Gegenstände (100) innerhalb der Behandlungskammer (2).

10. Verfahren zum Sterilisieren von Gegenständen (100) wie Kapseln, Vorformlingen, Behältern in einer Sterilisationseinheit (1) nach einem der vorhergehenden Ansprüche, wobei das Verfahren die folgenden Schritte umfasst:
Einspritzen von Plasma in eine Behandlungskammer (2);
Aufrechterhalten einer plasmagesättigten Umgebung innerhalb der Behandlungskammer (2), wobei der Schritt zum Aufrechterhalten einer plasmagesättigten Umgebung durch Erzeugen einer Saugluftbarriere mindestens an einem Einlassabschnitt (3) und einem Auslassabschnitt (4) der Behandlungskammer (2) erfolgt;
Bereitstellen eines kontinuierlichen Stroms von Gegenständen (100) über die Behandlungskammer (2), um sie in die plasmagesättigte Umgebung gelangen zu lassen und sie zu sterilisieren.

11. Verfahren zum Sterilisieren nach Anspruch 10, wobei die Gegenstände (100) die Behandlungskammer (2) durchqueren und einen linearen Vorschubweg beschreiben.

12. Verfahren zum Sterilisieren nach Anspruch 10, wobei die Gegenstände (100) die Behandlungskammer (2) durchqueren und einen zumindest teilweise krummlinigen Weg beschreiben.

## Revendications

1. Unité de stérilisation (1) pour stériliser des articles (100) tels que des capsules, paraisons, récipients, comprenant :
une chambre de traitement (2) comportant une section d'entrée (3) et une section de sortie (4) pour lesdits articles (100) ;
une zone de passage (20) pour le passage des articles (100) comportant une extension à l'intérieur de la chambre de traitement (2) de la section d'entrée (3) à la section de sortie (4) ;
des moyens d'alimentation en plasma (5, 6) pour fournir du plasma à ladite chambre de traitement (2) ;
un système de barrière d'air d'aspiration (7, 8) fonctionnellement actif sur la chambre de traitement (2) de manière à limiter l'entrée d'air de l'extérieur vers la chambre de traitement (2) et la sortie de plasma de la chambre de traitement (2) vers l'extérieur, maintenant ainsi un environnement saturé en plasma à l'intérieur de la chambre de traitement (2), ledit système de barrière d'air d'aspiration (7, 8) comprenant deux prises d'aspiration (7, 8) respectivement disposées en correspondance de la section d'entrée (3) et en correspondance de la section de sortie (4) de ladite chambre de traitement (2), ledit système de barrière d'air d'aspiration (7, 8) étant disposé à l'extérieur de la chambre de traitement (2) ;
une première ouverture (13) réalisée dans la section d'entrée (3) à travers laquelle les articles (100) sont alimentés à la chambre de traitement (2) et
une deuxième ouverture (14) réalisée dans la section de sortie (4) à travers laquelle les articles traités (100) sortent de la chambre de traitement (2), ledit système de barrière d'air d'aspiration (7, 8) étant disposé de manière à ne pas interférer avec lesdites ouvertures (13, 14).

2. Unité de stérilisation (1) selon l'une quelconque des revendications précédentes, dans laquelle lesdits moyens d'alimentation en plasma (5, 6) comprennent au moins un générateur de plasma (5) et une ligne de distribution de plasma (6) pour la distribution de plasma à la chambre de traitement (2).

3. Unité de stérilisation (1) selon la revendication 2, dans laquelle ladite ligne de distribution de plasma (6) se divise en au moins deux conduits (6a, 6b) pour l'injection de plasma dans la chambre de traitement (2).

4. Unité de stérilisation (1) selon la revendication 2 ou 3, dans laquelle ledit générateur de plasma (5) est une torche à plasma.

5. Unité de stérilisation (1) selon la revendication 1, dans laquelle lesdits moyens d'alimentation en plasma (5, 6) comprennent deux générateurs de plasma (5) disposés de manière à alimenter ladite chambre de traitement (2) à travers des lignes de distribution (6) correspondantes.

6. Unité de stérilisation (1) selon l'une quelconque des revendications précédentes, dans laquelle ladite zone de passage (20) pour le passage des articles (100) comporte une extension essentiellement linéaire à l'intérieur de la chambre de traitement (2).

7. Unité de stérilisation (1) selon l'une quelconque des revendications précédentes, dans laquelle ladite zone de passage (20) est délimitée par des parois de manière à définir un canal ou un tunnel (21) à l'intérieur de la chambre de traitement (2).

8. Unité de stérilisation (1) selon l'une quelconque des revendications 1 à 5, comprenant un carrousel rotatif pourvu d'une pluralité d'éléments de préhension pour saisir les articles (100) et disposé à l'intérieur de la chambre de traitement (2), ladite zone de passage (20) comporte une extension annulaire qui suit une trajectoire d'avancement circonférentielle des articles (100) pendant la rotation du carrousel rotatif.

9. Unité de stérilisation (1) selon l'une quelconque des revendications précédentes, comprenant un dispositif pour loger les articles (100) à l'intérieur de la chambre de traitement (2).

10. Procédé de stérilisation d'articles (100) tels que des capsules, paraisons, récipients dans une unité de stérilisation (1) selon l'une quelconque des revendications précédentes, ledit procédé comprenant les étapes de :
injecter du plasma dans une chambre de traitement (2) ;
maintenir un environnement saturé en plasma à l'intérieur de la chambre de traitement (2), l'étape consistant à maintenir un environnement saturé en plasma étant réalisée en générant une barrière d'air d'aspiration au moins en correspondance d'une section d'entrée (3) et d'une section de sortie (4) de la chambre de traitement (2) ;
fournir un flux continu d'articles (100) à travers la chambre de traitement (2) afin de les faire passer dans ledit environnement saturé en plasma et de les stériliser.

11. Procédé de stérilisation selon la revendication 10, dans lequel les articles (100) traversent la chambre de traitement (2) en décrivant une trajectoire d'avancement linéaire.

12. Procédé de stérilisation selon la revendication 10, dans lequel les articles (100) traversent la chambre de traitement (2) en décrivant une trajectoire au moins partiellement curviligne.
